# EUROPEAN PATENT SPECIFICATION

(11) **EP 3 599 264 B1**
(45) Date of publication and mention of the grant of the patent: **08.11.2023**
(21) Application number: 19195123.5
(22) Date of filing: 13.03.2014
(51) Int. Cl.: A61K 38/39, A61F 13/00, A61K 31/717, A61K 31/722, A61K 31/734

(54) **ADVANCED FUNCTIONAL BIOCOMPATIBLE POLYMER PUTTY USED AS A HEMOSTATIC AGENT FOR TREATING DAMAGED TISSUE AND CELLS**
VERBESSERTER KITT AUS FUNKTIONELLEM BIOKOMPATIBLEM POLYMER ALS HÄMOSTYPTIKUM ZUR BEHANDLUNG VON GESCHÄDIGTEM GEWEBE UND ZELLEN
PÂTE POLYMÈRE BIOCOMPATIBLE FONCTIONNELLE AVANCÉE UTILISÉE COMME AGENT HÉMOSTATIQUE POUR LE TRAITEMENT DE TISSU ET DE CELLULES ENDOMMAGÉS

(30) Priority: 13.03.2013 US 201361779706 P
(43) Date of publication of application: 29.01.2020
(62) Divisional of application: 14773437.0
(73) Proprietor: The University of Maryland, College Park, College Park, Maryland 20742 (US)
(72) Inventor: DIEHN, Kevin, Towson, MD 21286 (US); DOWLING, Matthew, College Park, MD Maryland 20742 (US); RAGHAVAN, Srinivasa R., Silver Spring, MD Maryland 20904 (US); KING, David R., Cambridge, MD Maryland 02114 (US)
(74) Representative: FRKelly

(56) References cited:
- WO-A1-2009/037500
- LIANG S ET AL: "Preparation of single or double-network chitosan/poly(vinyl alcohol) gel films through selectively cross-linking method", CARBOHYDRATE POLYMERS, APPLIED SCIENCE PUBLISHERS, LTD. BARKING, GB, vol. 77, no. 4, 19 July 2009 (2009-07-19), pages 718-724, XP026130488, ISSN: 0144-8617, DOI: 10.1016/J.CARBPOL.2009.02.007 [retrieved on 2009-02-24]
- MATTHEW B DOWLING ET AL: "A self-assembling hydrophobically modified chitosan capable of reversible hemostatic action", BIOMATERIALS, ELSEVIER SCIENCE PUBLISHERS BV., BARKING, GB, vol. 32, no. 13, 22 December 2010 (2010-12-22), pages 3351-3357, XP028366478, ISSN: 0142-9612, DOI: 10.1016/J.BIOMATERIALS.2010.12.033 [retrieved on 2010-12-29]
- KIM J O ET AL: "Development of polyvinyl alcohol-sodium alginate gel-matrix-based wound dressing system containing nitrofurazone", INTERNATIONAL JOURNAL OF PHARMACEUTICS, ELSEVIER, NL, vol. 359, no. 1-2, 9 July 2008 (2008-07-09), pages 79-86, XP022707701, ISSN: 0378-5173, DOI: 10.1016/J.IJPHARM.2008.03.021 [retrieved on 2008-03-22]
- PAUL A. MCCARRON ET AL: "Preliminary Clinical Assessment of Polyvinyl Alcohol-Tetrahydroxyborate Hydrogels as Potential Topical Formulations for Local Anesthesia of Lacerations : TOPICAL ANAESTHESIA USING PVA HYDROGELS", ACADEMIC EMERGENCY MEDICINE, vol. 18, no. 4, 15 April 2011 (2011-04-15) , pages 333-339, XP55377946, US ISSN: 1069-6563, DOI: 10.1111/j.1553-2712.2011.01032.x

## Description

### TECHNICAL FIELD

This invention relates to the field of polymer applications for the treatment of wounds.

### BACKGROUND ART

Around the turn of the new millennium, commercially available hemostatic technologies abruptly changed from ancient to new-age. While cotton gauze had served as the gold standard for treatment of bad bleeds for thousands of years, it was displaced by an arsenal of new materials which had been engineered to rapidly stop bleeding from severe injuries of the extremities. Such technologies were useful to US soldiers in both Operation Iraqi Freedom and Operation Enduring Freedom. The key products contracted to the military include 1) Quikclot^{®} (a registered trademark of Z-MEDICA, LLC) Combat Gauze, a gauze impregnated with kaolin nanoparticle powder which absorbs large amounts of water and hence concentrates clotting factors, 2) the Hemcon Patch^{®} (a registered trademark of HemCon Medical Technologies, Inc.), a freeze-dried bandage composed of chitosan, a material extracted from shrimp shells, and 3) Woundstat^{®} (a registered trademark of TraumaCure, Inc.), a clay mineral-based powder which, like Quikclot, absorbs high volumes of fluid quickly.

Although these products typically have done an adequate job of treating bleeding from extremities or superficial wounds in combat, they all have significant drawbacks. For example, Quikclot nanoparticles and Woundstat granules are very difficult to remove once an injured soldier arrives at a medical facility; this can cause permanent tissue scarring and/or peripheral clotting. Furthermore, these powders also pose a liability of blowing into the eyes of soldiers and medics in the field. The Hemcon Bandage is relatively easy to remove, but it offers little flexibility in application due to the preformed shape of the solid bandage. Combat Gauze, and other gauze types, requires significant manual packing and compression times which put the lives of field medics at significant risk, particularly when under fire. Indeed, the salient common denominator among all currently available hemostats is the requirement for sustained compression. It is also worth noting that even with sustained compression on the bleeding site, such products demonstrate only mediocre effectiveness in treating severe life-threatening bleeds on the battlefield.

Like the solid, compressible products (gauze, bandage, powder) described above, countless other related technologies have also been developed over the last decade using essentially the same form factors and raw materials, with only incremental (or ambiguous) improvements demonstrated in pre-clinical studies. As such, a need exists for a hemostatic material which is easily packable and requires no sustained compression time. Of course, this material also should be highly effective, safe, low cost, durable and easily removable for it to be useful to military and civilian emergency medics.

Kim et al. (2008) relates to polyvinyl alcohol (PVA)/sodium alginate (SA) hydrogel matrix-based wound dressing systems containing nitrofurazone (NFZ), a topical anti-infective drug.

### DESCRIPTION OF THE INVENTION

A composition of matter for use in healing wounds and stopping hemorrhage is described in this application. The composition of matter has four components: a hemostatic biopolymer, wherein the hemostatic biopolymer is hydrophobically modified alginate a secondary polymer, wherein the secondary polymer is polyvinyl alcohol, an ionic crosslinker, and a solvent. The crosslinker may be sodium borate, a weak crosslinker.

The hybrid composition of matter is for use in a method for controlling bleeding. The composition of matter may be part of a kit for application of the composition of matter to a wound.

### BRIEF DESCRIPTION OF THE DRAWINGS

The above and other features, aspects, and advantages of the present invention are considered in more detail, in relation to the following description of embodiments thereof shown in the accompanying drawings, in which:

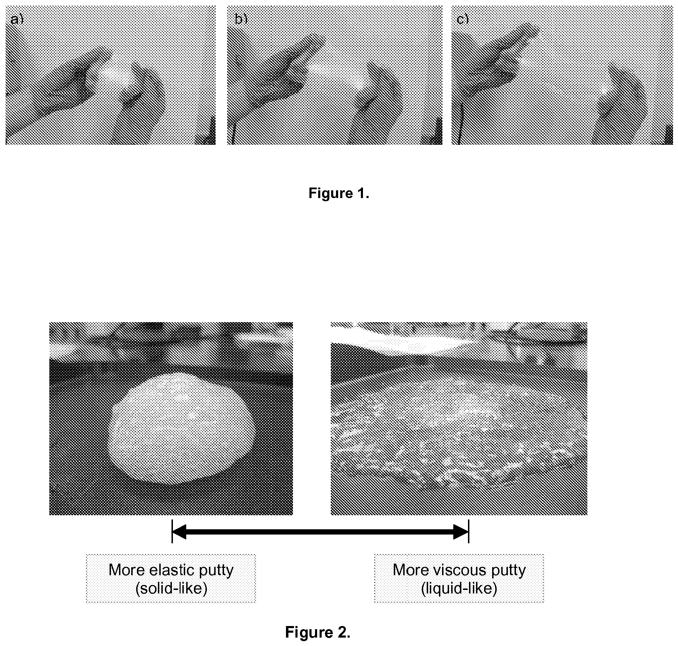

FIG 1 shows a demonstration of viscoelastic form factor of polyvinyl alcohol-borate-chitosan putty is shown. The putty is elastic (solid-like) enough to be held in the hand, but the putty is viscous (liquid-like) enough to be molded and stretched to various shapes to fit wounds and will conform to any wound topography.
FIG 2 shows an example putty (polyvinyl alcohol-borate-chitosan) can be tuned to have specific viscous and elastic properties by modifying polymer concentration, polymer molecular weight, crosslinker type, and crosslinker concentration. Here the polymer concentration is varied from about 12 wt % (a) to 7 wt% (b) with a borate crosslinker at a 1:40 mass ratio of the polymer. The chitosan concentration is 1 wt% in both 2 (a) and 2 (b).

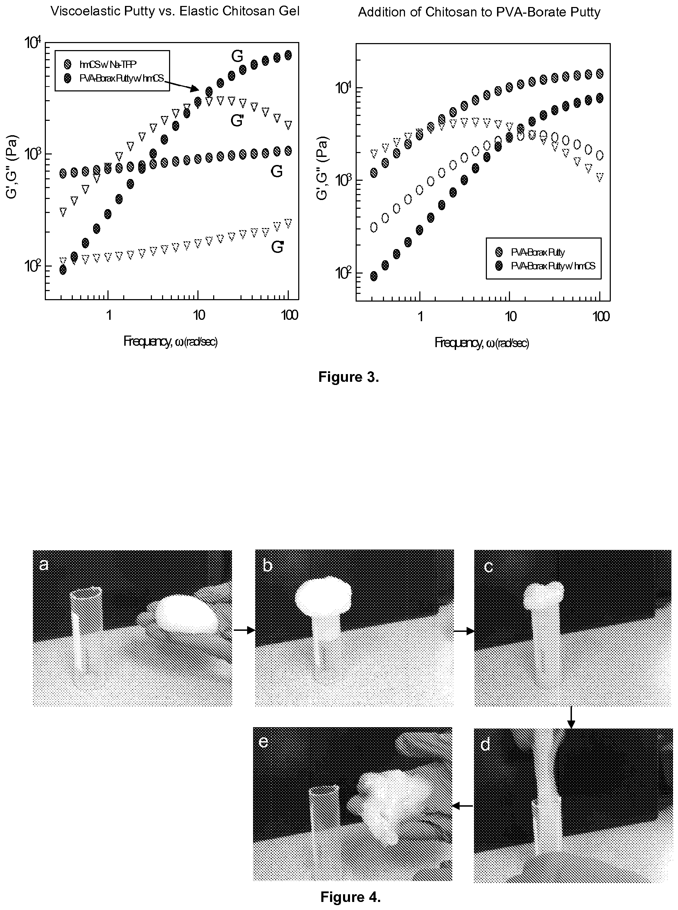

FIG 3 shows (a) dynamic rheology of a viscoelastic putty consisting of polyvinyl alcohol crosslinked with sodium borate (PVA-Borax) blended with hydrophobically-modified chitosan (hmCS) (upper curves) vs. an hmCS sample crosslinked with sodium tripolyphosphate (NaTPP) (lower curves) and (b) dynamic rheology of a polyvinyl alcohol-borate putty (upper curves) and of a PVA-Borax putty with hemostatic polymer, hmCS, added (lower curves).
FIG 4 shows an example putty, polyvinyl alcohol crosslinked with sodium borate (PVA-Borax) blended with hydrophobically-modified chitosan (hmCS), (a) can be easily inserted into a simulated three-dimensional wound (b), holds its own weight (c), and be removed entirely from the cavity (d-e).

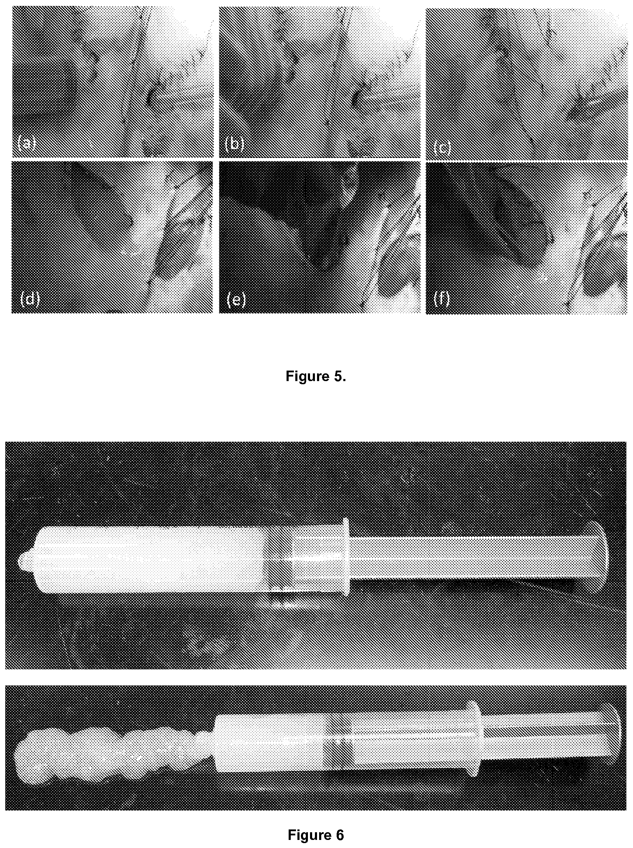

FIG 5 shows an *in vivo* application of hemostatic putty to porcine arterial 5-French laceration (gunshot model). In (a), a 60 cc syringe loaded with putty, consisting of polyvinyl alcohol crosslinked with sodium borate (PVA-Borax) blended with hydrophobically-modified chitosan (hmCS), is positioned near an aperture above the artery. In (b), the putty is syringed into the bleeding cavity through the aperture. In (c), the delivered putty is observable at the aperture. Next in (d), the aperture is widened to observe hemostasis in the cavity filled with putty. Finally, in (e) and (f) the putty is removed by hand, with no re-bleeding observed.
FIG 6 shows a diagram of a syringe wherein the composition of matter is contained as a kit for application to a wound.

### BEST MODE(S) FOR CARRYING OUT THE INVENTION

The invention summarized above may be better understood by referring to the following description, which should be read in conjunction with the accompanying claims and drawings in which like reference numbers are used for like parts. This description of an embodiment, set out below to enable one to build and use an implementation of the invention, is not intended to limit the invention, but to serve as a particular example thereof.

### Hybrid Composition of Matter of Putty

In this application the term "hemostat" means a composition of matter, such as a polymer putty, that adheres to tissues and forms aggregates with blood cells and blood cell clots, assisting in the clotting process and effectively stopping hemorrhage. As utilized here, the term "aggregate" refers to the hydrophobic interactions between the red blood cells and a hydrophobically modified biopolymer and interactions with other components of the composition of matter that assist in the control of hemorrhage.

The term "hydrophobically modified biopolymer" refers to a synthetic or naturally occurring polysaccharide or peptide; where the hydrophobic modification consists of the covalent binding of hydrophobic substituents to the polymer backbone. The manufacture and composition of the hydrophobically modified biopolymer is described in United State Patents 8,664,199 and 8,668,899; and also in United States Application Publication Numbers US2008/0254104A1, US2009/0062849A1, and US2012/0252703A1.

A hybrid composition of matter that provides a moldable and adhesive hemostatic agent comprises at least four components: (1) a hemostatic biopolymer, wherein the hemostatic biopolymer is hydrophobically modified alginate; (2) a secondary polymer matrix, wherein the secondary polymer is polyvinyl alcohol; (3) an ionic crosslinker, and (4) a solvent. The combination of these elements forms a "putty," which in this application refers to a composition of matter with sufficient viscosity and hemostatic activity to assist in controlling bleeding and hemorrhage.

The hemostatic biopolymer comprises a hydrophobically modified alginate capable of hydrophobically interacting with tissue, particularly hemostatic interaction with damaged tissue. In some embodiments, the hemostatic polymer is comprised of at least one polymer and a plurality of short hydrophobic substituents attached along the backbone of the polymer. The polymer is either synthetic or naturally occurring, including for example, water-soluble polysaccharides and water-soluble polypeptides. For example, the polymer is one or more hydrophobically modified alginates.

All four types of materials allow for the transfer of oxygen and moisture required to metabolize the wound healing physiology. Chitosan also has inherent anti-microbial properties; this is a crucial asset for materials covering open wounds because it eliminates the need to constantly change wound dressings in order to disinfect the wound manually between changes. Positive charges along the backbone of chitosan cause it to interact electrostatically with negatively charged blood cells, thus creating a sticky interface between the putty and the wound. Chitosan provides hemostasis for a period of about 30 min before becoming saturated with blood cells and losing adhesion to the wound site. Hydrophobically-modified (hm) - chitosan, in particular, has been shown to increase the tissue adhesion properties of materials in which it is incorporated. Here, too, the addition of hm-chitosan into a PVA-Borax putty increases the overall adhesive properties of the putty. Adhesiveness of the putties can be tuned by the amount of hm-chitosan included in the blend relative to PVA-Borax, or by the level of hydrophobic modified to the hm-chitosan molecule itself.

Chitosans (whether modified or unmodified) include, for example, the following chitosan salts: chitosan lactate, chitosan salicylate, chitosan pyrrolidone carboxylate, chitosan itaconate, chitosan niacinate, chitosan formate, chitosan acetate, chitosan gallate, chitosan glutamate, chitosan maleate, chitosan aspartate, chitosan glycolate and quaternary amine substituted chitosan and salts thereof, etc. Alginates (whether modified or unmodified) include, for example, sodium alginate, potassium alginate, magnesium alginate, calcium alginate, aluminum alginate, etc. Cellulosics include, for example, hydroxyetyhl cellulose, hydroxypropyl cellulose, methyl cellulose, hydroxypropyl methyl cellulose, hydroethyl methyl cellulose, etc. In an example, the polymeric component of the hemostat comprises mixtures of polysaccharides between classes, e.g., cellolusics and chitosans, or within the same class, e.g., two different types of alginate.

The polymeric components suitable for use in the hybrid composition can comprise one or more hydrophobically modified alginates. Such polysaccharide starting materials from which the hydrophobically modified polysaccharides can be made are known to those skilled in the art. Cellulosics, chitosans and alginates are all abundant, natural biopolymers. Cellulosics are found in plants, whereas chitosans and alginates are found in the exoskeleton or outer membrane of a variety of living organisms. All three types of materials allow for the transfer of oxygen and moisture required to metabolize the wound healing physiology.

Gelatins are polypeptides derived from the skin and bones of mammals. These polypeptides are degraded forms of their pre-cursor protein, collagen. Gelatin is a thermo-reversible biopolymer which undergoes a phase transition from solid to liquid above 32°C at concentrations of 3 wt% or greater in water. Sources of gelatin include gelatin from bovine skin, gelatin from bovine bones, gelatin from porcine skin and gelatin from porcine bones. These polypeptides display excellent biocompatibility and resorption characteristics.

The second component of the putty is a polyvinyl alcohol polymer matrix. In a preferred embodiment, the secondary polymer matrix is a polyvinyl alcohol (PVA) in the molecular weight range of 125,000 Da to 140,000 Da. In a further embodiment, the PVA is in the molecular weight range of 50,000 Da to 125,000 Da. In another further embodiment, the PVA is in the molecular weight range of 140,000 Da to 300,000 Da. In such exemplary embodiment, the secondary polymer can be included in concentration of between 5 wt% and 15 wt%. In a preferred embodiment, the level of hydrolysis of the PVA is in a range from 70% to 100% hydrolyzed. In a further embodiment, the level of hydrolysis of the PVA is in a range from 30% to 70%. A crosslinker, sodium tetraborate decahydrate, is included in the blend at a concentration of between 0.1 wt% and 1.0 wt%. The hemostatic polymer, chitosan or hydrophobically-modified chitosan, is included in the putty blend a concentration of between 0.1 wt% and 3 wt%. It is contemplated that the crosslinker may include sodium tetraborate decahydrate, or other borate salts, such as sodium perborate or sodium metaborate.

The crosslinker interacts with the secondary polymer based upon hydrogen bonding or ionic weak interactions. For example, when borate salts are used as a crosslinker, di-diol interactions with the matrix polymer assist in enhancing the viscoelasticity of the hybrid composition. Such interactions are present in compositions without a crosslinker, but addition of the crosslinker helps enhance these properties. The weak interaction between the polymer chains results in the viscoelastic character of the putty that enables it to conform to highly three-dimensional wounds. Likewise, the viscoelastic character may be tuned by varying polymer molecular weight, polymer concentration, crosslinker type, or concentration of crosslinker. The putty may be comprised of entirely matrix polymer with the hemostatic polymer as an additive or it may be comprised of the matrix polymer in a solvent, for example water or organic solvents.

The ratio of the liquid and solid-like character of the putty can be tuned through the use of different polymer concentrations, polymer molecular weights, type of ionic cross linker, or concentration of ionic cross linker. For example, the putty may be in the form of a very viscous fluid, but one that is still capable of being injected from a medical syringe into very narrow wounds, such as gunshot or impalement wounds. Likewise, the putty may take the form of a higher modulus, solid-like pad for shallower wounds. Note that the solid-like putty will still deform over time to conform to the three-dimensional topography of the wound. Additionally, the weak crosslinking nature of the putty enables it to swell when in contact with wound exudate. This swelling action of the putty can provide a compressive force normal to the wound surface when placed into a confined wound cavity. In a preferred embodiment the compressive force is between 5 and 15 N. In a further embodiment the compressive force is between 15 and 30 N. In a further preferred embodiment, the compressive force is between 0.1 and 5 N.

The weakly cross-linked polymer matrix has both *viscous* properties of a liquid (i.e. it flows if left for long times on a surface) and *elastic* properties of a solid (i.e. it can be bounced off of a surface like a rubber ball) and thus can be termed viscoelastic (having both liquid and solid character). This viscoelastic character enables the putty to conform to the three-dimensional topography of wounds to ensure intimate contact of the hemostatic polymer agent and the surface of the wound. Likewise, the solid-like nature of the material enables the putty to transmit external compressive force to the interior surfaces of the wound.

The fourth component of the hybrid composition of matter consists of a solvent. In some preferred embodiments, the solvent is water.

In one preferred embodiment, the composition of matter, putty, is composed of a (1) hydrophobically modified alginate matrix capable of hydrophobically interacting with tissue, particularly hemostatic interaction with damaged tissue, (2) polyvinyl alcohol (PVA), (3) a borate salt, such as sodium tetraborate decahydrate, and (4) water.

### Method of Manufacturing Hybrid Composition of Matter of Putty

The putty, or hybrid composition, is manufactured by adding the hemostatic polymer to the secondary matrix polymer, in some cases one or both may be dissolved in solvent, and adding the crosslinker to the polymer mixture. In a preferred embodiment, the secondary matrix polymer is PVA. The PVA is dissolved in water by heating at 95°C for 15 minutes under stirring. The PVA is then cooled to room temperature and mixed with the hemostatic polymer and the crosslinker. This mixture is then stirred or kneaded for 15 minutes to 60 minutes to mix the components and entangle the matrix polymer chains until yielding a viscoelastic putty.

In another embodiment, the putty material may be made of such viscosity that it can be used as a hemostatic pad for topical application on wounds. The putty should be soft enough that it can be shaped to appropriate size and may conform closely to the three dimensional topography of the wound, but solid enough that it can withstand the force of compression from the individual administering treatment to the wound and that it can transmit this compressive force to the surface of the wound.

In one exemplary method, the hydrophobically modified alginate is manufactured, where the hydrophobic modifications consist of the addition of hydrophobic substituents to the polymer's backbone. In one example, a hydrophobic substituent comprising a hydrocarbon group having from about 8 to about 18 carbon atoms is attached to the backbone of the at least one alginate polysaccharide. In an example, the hydrocarbon group comprises an alkyl or arylalkyl group. As used herein, the term "arylalkyl group" means a group containing both aromatic and aliphatic structures. Procedures for hydrophobically modifying the above mentioned polysaccharides are described in United State Patents 8,664,199 and 8,668,899; and also in United States Application Publication Numbers US2008/0254104A1, US2009/0062849A1, and US2012/0252703A1.

In one embodiment of the present hemostatic composition, the degree of substitution of the hydrophobic alginate substituent on the polysaccharide is from about 1 to about 100 moles of the hydrophobic alginate substituent per mole of the polysaccharide. In an example, more than one particular hydrophobic substituent is substituted onto the alginate polysaccharide, provided that the total substitution level is substantially within the ranges set forth above. In a further preferred embodiment, the degree of substitution of the hydrophobic alginate substituent on the polysaccharide is from about 40 to 65 moles of the hydrophobic alginate substituent per mole of the polysaccharide. In another preferred embodiment, the molecular weight of the alginate polysaccharides used as the hemostatic polymer range from about 50,000 to about 1,500,000 grams per mole. In examples, the molecular weight of the polysaccharides comprising the spray ranges from about 50,000 to about 25,000 grams per mole. As used herein, the term "molecular weight" means weight average molecular weight. The preferred methods for determining average molecular weight of polysaccharides are low angle laser light scattering (LLS) and Size Exclusion Chromatography (SEC). In performing low angle LLS, a dilute solution of the polysaccharide, typically 2% or less, is placed in the path of a monochromatic laser. Light scattered from the sample hits the detector, which is positioned at a low angle relative to the laser source. Fluctuation in scattered light over time is correlated with the average molecular weight of the polysaccharide in solution. In performing SEC measurements, again a dilute solution of polysaccharide, typically 2% or less, is injected into a packed column. The polysaccharide is separated based on the size of the dissolved polysaccharide molecules and compared with a series of standards to derive the molecular weight.

In an example, the putty material is mixed with a variety of water-soluble reagents that result in faster and more efficient healing of the wound. A first class of reagents that is mixed with the hydrophobically modified polysaccharide is comprised of those reagents that contribute to the hemostatic integrity of the clot such as for example human thrombin, bovine thrombin, recombinant thrombin, and any of these thrombins in combination with human fibrinogen. Other examples of the first class of reagents include fibrinogen, Factor VIIa, and Factor XIII. A second class of reagents that is mixed with the hydrophobically modified polysaccharide is comprised of those reagents that prevent microbial infection such as norfloxacin (anti-microbial), silver (anti-microbial), ampicillin (antibiotic) and penicillin (antibiotic) Reagents from both classes, e.g. recombinant thrombin and norfloxacin, or reagents from the same class, e.g. recombinant thrombin and fibrinogen, may be mixed with the polysaccharide.

The hydrophobically modified alginate is combined with the polyvinyl alcohol secondary polymer matrix and a solvent. In a preferred embodiment, the secondary polymer matrix is a polyvinyl alcohol (PVA) in the molecular weight range of 125,000 Da to 140,000 Da. In such exemplary embodiment, the secondary polymer can be included in concentration of between 5 wt% and 15 wt%. The level of hydrolysis of the PVA may range from 70% to 100% hydrolyzed. A crosslinker, sodium tetraborate decahydrate, is included in the blend at a concentration of between 0.1 wt% and 1.0 wt%. The hemostatic polymer, alginate or hydrophobically-modified alginate, is included in the putty blend a concentration of between 0.1 wt% and 3 wt%.

### Methods of Utilizing the Hybrid Composition of Matter of Putty

In one embodiment of the present invention, a hemostatic composition and delivery system is used for treating both compressible and non-compressible hemorrhages effectively. Many different types of materials and syringe devices can be used. For surgical or medical field use, however, it can be desirable to sterilize the composition and the syringe device using autoclave, gamma-irradiation, electron beam irradiation or sterile ultrafiltration.

The composition can be used in a method for treating compressible and non-compressible hemorrhages through use of a hemostat. The putty is applied to a wound to stop bleeding and hemorrhage. The hemostatic putty, or polymer dough, is suitable for use in mammals. As used herein, the term "mammals" means any higher class of vertebrates that nourish their young with milk secreted by mammary glands, e.g. humans, rabbits and monkeys.

It is contemplated that this putty may be included as part of a syringe delivery system as may be known by those skilled in the art. The hemostatic interaction occurs through application of the moldable putty into an injured tissue site and subsequent process wherein a plurality of short hydrophobic substituents, that are attached with the biopolymer backbone, interact with and adhere to blood and tissue upon swelling of the putty.

In another embodiment, the putty material may be made of such viscosity that it may be pumped through a syringe into a deep wound cavity, such as a gunshot wound or impalement wound. The syringe here refers to any device that applies pressure to the viscoelastic fluid such that is flows out of the narrow tip of the device, thus enabling application of the putty to the wound. The putty, having been applied the wound, may be left alone to swell in the wound or may receive additional compression from the external part of the wound to aid in clot formation. After achieving hemostasis, the putty may be removed from the cavity as a single wound dressing

### Kits Comprising the Hybrid Composition of Matter of Putty

Also disclosed is a kit for treating wounds comprising a container with a hybrid composition described herein and an applicator. The applicator can be a syringe or any other type of apparatus known to a person of ordinary skill in the art to apply a semiliquid to an intended area, such as a wound. In one example, the kit consists of a pre-packed syringe containing a particular formulation of the putty and ready for use as shown in Figure 6.

### Example 1.

As shown in Figure 1, the hybrid composition putty described here has viscoelastic properties that are useful in treating acute wounds. The Figure shows how the composition can be manipulated easily to change its shape and be stretched significantly. The example in Figure 2 shows the change in elasticity of the putty depending on the concentration of polymer used. Here the polymer concentration is varied from about 12 wt % (a) to 7 wt% (b) with a borate crosslinker at a 1:40 mass ratio of the polymer. The chitosan concentration is 1 wt% in both 2 (a) and 2 (b).

Rheological Experiments: Steady shear rheological experiments were performed on a Rheometrics AR2000 stress-controlled rheometer. A cone-and-plate geometry of 40 mm diameter and 4° cone angle was used and samples were run at the physiological temperature of 37°C. Data on the same samples via dynamics rheology are shown in Figure 3 where the elastic (G') and viscous (G") moduli are plotted as a function of frequency (rad/s) of oscillatory shear. Here, we note that the putty sample has a transition in elastic-like behavior (G'>G") to viscous-like behavior (G" > G') over long time scales.

The putty or hybrid composition of described in here has been shown to work in simulated three-dimensional wounds as shown in Figure 4. The putty has been shown to be easily applied and removed from such simulated wounds. The putty has also been used in vivo as shown in Figure 5. The experimental design for this in vivo application is as follows. Female Yorkshire pigs, weighing 39.2 ± 2.3 kg (n=3) were obtained from the Knight Surgical Lab at Massachusetts General Hospital (Boston, MA). All animals were maintained in a facility accredited by the Association for Assessment and Accreditation of Laboratory Animal Care, and all experiments were performed in accordance with the National Research Council's Guide for the Care and Use of Laboratory Animals. The protocol was approved by the IACUC at Massachusetts General Hospital. The swine were anesthetized with 6 mg/kg of telazol and 0.01 mg/kg of glycopyrrolate that were given intramuscularly. They were incubated and placed on mechanical ventilation at a tidal volume 12 mL/kg, a rate of 10 respirations per minute, and 100% oxygen. Anesthesia was maintained using isoflurane, and ventilatory parameters were maintained to attain an end-tidal CO₂ partial pressure of 40 mm Hg.

To create a hemorrhage in the groin area, approximately 5 cm of the right femoral artery was dissected free from surrounding tissues and the overlying abductor muscle was removed. A 5 French catheter was inserted into the artery. Next, the skin above the artery was sutured closed, with the exception of a small aperture approximately 2 cm in diameter. Finally, the catheter was pulled from the artery to create hemorrhage.

In (a), a 60 cc syringe loaded with putty, consisting of polyvinyl alcohol crosslinked with sodium borate (PVA-Borax) blended with hydrophobically-modified chitosan, is positioned near an aperture above the femoral artery. Once the catheter was removed from the artery, significant bleeding within the cavity occurred. A small hole in the skin remained the only access point to the bleed. Compressible devices such as bandages and gauze were not able to be applied through the hole. In (b), the putty is syringed into the bleeding cavity through the aperture after 1 minute of free bleeding. In (c), the delivered putty is observable at the aperture. Next in (d), the aperture is widened to observe hemostasis in the cavity filled with putty. Finally, in (e) and (f) the putty is removed by hand after 30 min, with no re-bleeding observed.

The invention has been described with references to a preferred embodiment. While specific values, relationships, materials and steps have been set forth for purposes of describing concepts of the invention, it will be appreciated by persons skilled in the art that numerous variations and/or modifications may be made to the invention as shown in the specific embodiments within the scope of the claims. It should be recognized that, in the light of the above teachings, those skilled in the art can modify those specifics within the scope of the claims. Having now fully set forth the preferred embodiments and certain modifications of the concept underlying the present invention, various other embodiments as well as certain variations and modifications of the embodiments herein shown and described will obviously occur to those skilled in the art upon becoming familiar with such underlying concept. It is intended to include all such modifications, alternatives and other embodiments insofar as they come within the scope of the appended claims. Consequently, the present embodiments are to be considered in all respects as illustrative and not restrictive.

### INDUSTRIAL APPLICABILITY

The present invention is applicable to wound dressings and drug delivery. A composition of matter is disclosed having a hemostatic biopolymer, a secondary biopolymer, an ionic crosslinker and a solvent and a method for its use. The composition of matter can be made in industry and practiced in the wound treatment field.

## Claims

1. A putty, comprising:
a hemostatic biopolymer, wherein the hemostatic biopolymer is hydrophobically modified alginate;
a secondary polymer, wherein the secondary polymer is polyvinyl alcohol;
an ionic crosslinker; and
a solvent.

2. The putty of claim 1, wherein the hydrophobically modified biopolymer comprises hydrophobic substituents covalently attached to the biopolymer.

3. The putty of claim 2, wherein the hydrophobic substituents comprise from eight to eighteen carbon atoms; optionally wherein the hydrophobic modification is of 1 to 100 moles of hydrophobic substituent per 1 mole of biopolymer.

4. The putty of claim 1, wherein the hydrophobically modified alginate is selected from the group consisting of sodium alginate, potassium alginate, magnesium alginate, calcium alginate, aluminum alginate.

5. The putty of claim 1, wherein the polyvinyl alcohol is present in a concentration of between 5wt% and 15wt%;

6. The putty of claim 5, wherein the polyvinyl alcohol has a molecular weight in of between 50,0000a and 300,000Da.

7. The putty of claim 5, wherein the polyvinyl alcohol is hydrolyzed in a range of 70% to 100%.

8. The composition of matter of claim 1, wherein the crosslinker is present in a concentration of between 0.1wt% and 1.0wt%.

9. The putty of claim 1, wherein the hemostatic biopolymer is present in a concentration of between 0.1wt% and 3.0wt%.

10. The putty of claim 1, wherein the composition of matter is capable of providing a compressing force of between 5 and 15 N, 15 and 30 N, or 0.1 and 5N.

11. The putty of claim 5, wherein the polyvinyl alcohol has a molecular weight in of between 125,000 Da and 140,000 Da.

12. The putty of claim 1, where in the crosslinker is a borate salt; optionally wherein the borate salt is selected from the group consisting of sodium tetraborate decahydrate, sodium peroborate, and sodium metaborate.

13. The putty of claim 1, further comprising a water soluble reagent; optionally wherein the water soluble reagent is selected from the group consisting of human thrombin, bovine thrombin and recombinant thrombin.

14. The putty of claim 1, further comprising a reagent selected from the group consisting of fibrinogen, factor Vila, Factor XIII.

15. The putty of claim 1, further comprising a reagent that prevent microbial infections.

## Patentansprüche

1. Kitt, umfassend:
ein hämostyptisches Biopolymer, wobei das hämostyptische Biopolymer hydrophob modifiziertes Alginat ist;
ein sekundäres Polymer, wobei das sekundäre Polymer Polyvinylalkohol ist;
einen ionischen Vernetzer; und
ein Lösungsmittel.

2. Kitt nach Anspruch 1, wobei das hydrophob modifizierte Biopolymer an das Biopolymer kovalent gebundene hydrophobe Substituenten umfasst.

3. Kitt nach Anspruch 2, wobei die hydrophoben Substituenten 8 bis 18 Kohlenstoffatome umfassen; wobei optional die hydrophobe Modifikation 1 bis 100 Mol hydrophober Substituent pro 1 Mol Biopolymer beträgt.

4. Kitt nach Anspruch 1, wobei das hydrophob modifizierte Alginat ausgewählt ist aus der Gruppe bestehend aus Natriumalginat, Kaliumalginat, Magnesiumalginat, Calciumalginat, Aluminiumalginat.

5. Kitt nach Anspruch 1, wobei der Polyvinylalkohol in einer Konzentration zwischen 5 Gew.-% und 15 Gew.-% vorliegt.

6. Kitt nach Anspruch 5, wobei der Polyvinylalkohol ein Molekulargewicht zwischen 50.000 Da und 300.000 Da aufweist.

7. Kitt nach Anspruch 5, wobei der Polyvinylalkohol in einem Bereich von 70% bis 100% hydrolysiert ist.

8. Stoffzusammensetzung nach Anspruch 1, wobei der Vernetzer in einer Konzentration zwischen 0,1 Gew.-% und 1,0 Gew.-% vorliegt.

9. Kitt nach Anspruch 1, wobei das hämostyptische Biopolymer in einer Konzentration zwischen 0,1 Gew.-% und 3,0 Gew.-% vorlieg.

10. Kitt nach Anspruch 1, wobei die Stoffzusammensetzung in der Lage ist, eine Presskraft zwischen 5 und 15 N, 15 und 30 N oder 0,1 und 5 N bereitzustellen.

11. Kitt nach Anspruch 5, wobei der Polyvinylalkohol ein Molekulargewicht zwischen 50.000 Da und 300.000 Da aufweist.

12. Kitt nach Anspruch 1, wobei in dem Vernetzer ein Boratsalz ist; wobei optional das Boratsalz ausgewählt ist aus der Gruppe bestehend aus Natriumtetraboratdekahydrat, Natriumperborat und Natriummetaborat.

13. Kitt nach Anspruch 1, ferner umfassend ein wasserlösliches Reagenz; wobei optional das wasserlösliche Reagenz ausgewählt ist aus der Gruppe bestehend aus humanem Thrombin, Rinderthrombin und rekombinantem Thrombin.

14. Kitt nach Anspruch 1, ferner umfassend ein Reagenz ausgewählt aus der Gruppe bestehend aus Fibrinogen, Faktor VIIa, Faktor XIII.

15. Kitt nach Anspruch 1, ferner umfassend ein Reagenz, das mikrobiellen Infektionen vorbeugt.

## Revendications

1. Une pâte, comprenant :
un biopolymère hémostatique, dans laquelle le biopolymère hémostatique est de l'alginate modifié hydrophobiquement ;
un polymère secondaire, le polymère secondaire étant un alcool polyvinylique ;
un réticulant ionique ; et
un solvant.

2. Pâte selon la revendication 1, **caractérisée en ce que** le biopolymère modifié hydrophobiquement comprend des substituants hydrophobes liés de manière covalente au biopolymère.

3. Pâte selon la revendication 2, dans laquelle les substituants hydrophobes comprennent de huit à dix-huit atomes de carbone ; éventuellement dans laquelle la modification hydrophobe est de 1 à 100 moles de substituant hydrophobe pour 1 mole de biopolymère.

4. Pâte selon la revendication 1, dans laquelle l'alginate modifié hydrophobiquement est choisi parmi le groupe constitué de l'alginate de sodium, l'alginate de potassium, l'alginate de magnésium, l'alginate de calcium, l'alginate d'aluminium.

5. Pâte selon la revendication 1, dans laquelle l'alcool polyvinylique est présent à une concentration comprise entre 5 % et 15 % en masse ;

6. Pâte selon la revendication 5, dans laquelle l'alcool polyvinylique présente une masse moléculaire comprise entre 50 000 Da et 300 000 Da.

7. Pâte selon la revendication 5, dans laquelle l'alcool polyvinylique est hydrolysé dans une plage de 70 % à 100 %.

8. Composition de matière selon la revendication 1, **caractérisée en ce que** l'agent réticulant est présent à une concentration comprise entre 0,1 % et 1,0 % en masse.

9. Pâte selon la revendication 1, dans laquelle le biopolymère hémostatique est présent à une concentration comprise entre 0,1 % et 3,0 % en masse.

10. Pâte selon la revendication 1, dans laquelle la composition de matière est apte à fournir une force de compression comprise entre 5 et 15 N, 15 et 30 N, ou 0,1 et 5 N.

11. Pâte selon la revendication 5, dans laquelle l'alcool polyvinylique présente un poids moléculaire compris entre 125000 Da et 140 000 Da.

12. Pâte selon la revendication 1, dans laquelle l'agent réticulant est un sel de borate ; éventuellement dans laquelle le sel de borate est choisi parmi le groupe constitué par le tétraborate de sodium décahydraté, le perborate de sodium et le métaborate de sodium.

13. Pâte selon la revendication 1, comprenant en outre un réactif soluble dans l'eau, éventuellement dans laquelle le réactif soluble dans l'eau est choisi parmi le groupe constitué de la thrombine humaine, de la thrombine bovine et de la thrombine recombinante.

14. Pâte selon la revendication 1, comprenant en outre un réactif choisi parmi le groupe constitué du fibrinogène, du facteur VIIa, du facteur XIII.

15. Pâte selon la revendication 1, comprenant en outre un réactif qui prévient les infections microbiennes.
